# EUROPÄISCHE PATENTSCHRIFT

(11) **EP 2 094 250 B1**
(45) Veröffentlichungstag und Bekanntmachung des Hinweises auf die Patenterteilung: **28.04.2010**
(21) Anmeldenummer: 07846633.1
(22) Anmeldetag: 16.11.2007
(51) Int. Cl.: A61K 9/70, A61K 47/32

(54) **TRANSDERMALES THERAPEUTISCHES SYSTEM MIT IONENPAAR-MIKRORESERVOIREN**
TRANSDERMAL THERAPEUTIC SYSTEM COMPRISING ION PAIR MICRO-RESERVOIRS
SYSTÈME THÉRAPEUTIQUE TRANSDERMIQUE À MICRORÉSERVOIRS DE PAIRES D'IONS

(30) Priorität: 21.11.2006 DE 102006054732
(43) Veröffentlichungstag der Anmeldung: 02.09.2009
(73) Patentinhaber: LTS LOHMANN Therapie-Systeme AG, 56626 Andernach (DE)
(72) Erfinder: HILLE, Thomas, 56073 Koblenz (DE); HORSTMANN, Michael, 56564 Neuwied (DE); MÜLLER, Walter, 56626 Andernach (DE)
(74) Vertreter: Schmidt, Werner
(86) Internationale Anmeldenummer: PCT/EP2007/009926
(87) Internationale Veröffentlichungsnummer: WO 2008/061677

(56) Entgegenhaltungen:
- EP-A- 0 387 647
- WO-A-01/01967
- WO-A-02/41878
- DE-A1- 10 012 908
- DE-A1-102005 010 255

## Beschreibung

Transdermale Therapeutische Systeme (TTS) sind seit einer Reihe von Jahren bekannt. Solche Systeme sind z. B. für Wirkstoffe wie Estradiol, Norethisteronacetat, Nikotin, Fentanyl, Tolubuterol, Acetyl-Salicylsäure, Buprenorphin und eine Reihe weiterer Wirkstoffe in die Therapie eingeführt. Dabei kann man die pharmazeutisch geeigneten Wirkstoffe in die Gruppe der Neutralstoffe (Norethisteronacetat, Estradiol, Nitroglycerin etc.), der sauren Wirkstoffe (Acetylsalicylsäure, Ketoprofen, Ibuprofen etc.) und insbesondere der basischen Wirkstoffe (Nikotin, Fentanyl, Tolubuterol, Buprenorphin, Moxonidin, Selegilin, Salbutamol und viele andere) unterteilen.

Als "saure" Wirkstoffe bzw. Verbindungen sollen hier solche verstanden werden, die elektrophil sind und somit als Elektronenpaarakzeptoren (Anionenbildner) reagieren können, als "basische" Wirkstoffe bzw. Verbindungen solche, die nucleophil sind und somit als Elektronenpaardonatoren (Kationenbildner) reagieren können. Beispiele für die erstgenannten sind Neutralsäuren (z.B. HCL), Kationsäuren, (z.B. NH₄⁺) oder Anionsäuren (z.B. HCO₃⁻). Beispiele für die basischen Stoffe sind Neutralbasen (z.B. NH3 und organische Amine), Kationbasen (z.B. NH₂-NH₃⁺) und Anionbasen (z.B. CLO₄⁻).

Der Aufbau von transdermalen therapeutischen Systemen ist in der Regel dünn und geschichtet, so daß sich mit Hilfe der direkt der Haut zugewandten Seite (Klebschicht) eine zumindest vorübergehend klebrige Verbindung zur Haut ergibt, über die der Wirkstoff abgegeben wird. Zur Verbesserung der Wirkstoffpermeation durch die Haut werden neben Polymeren, Harzen und anderen pharmazeutischen Hilfsstoffen auch flüssige Systemkomponenten eingesetzt, die im engeren Sinn als Permeationsverstärker bekannt sind. Ihr Ziel ist die Verbesserung des Wirkstofftransportes durch die Haut. Dabei können Permeationsverstärker ihre Wirkung rein physikalisch dadurch ausüben, daß sie die Diffusion des Wirkstoffes innerhalb des pharmazeutischen Systems verbessern, oder aber dadurch, daß sie gleichzeitig Lösungsverbesserer sind und nach Diffusion in die Haut dort eine thermodynamische Aktivierung auslösen (EP 1 191 927 B) oder aber (Enhancer im engeren Sinn) durch direkten Einfluß auf die Diffusibilität in der Haut eine Erhöhung der Substanzzufuhr an den Körper ermöglichen. Innerhalb dieser Enhancer ist in chemischer Hinsicht eine Untergruppe abzugrenzen, die sich als "Ionenpaarbildner" beschreiben läßt. Es sind dies im Falle der vorher erwähnten sauren Wirkstoffe physiologisch unbedenkliche nucleophile basische Zusatzstoffe, wie z.B. Ammoniak, Propylamin, Tromethamol, Triethanolamin u.v.a. aus der Literatur bekannte Stoffe. Im Falle der häufiger eingesetzten basischen Wirkstoffe werden saure, d.h. elektrophile Zusatzstoffe wie z.B. Ölsäure, Laurinsäure, Laevulinsäure und andere Fettsäuren, aber auch aromatische Säuren wie Benzoesäure oder auch Sulfonsäuren wie z.B. Hexansulfonsäure zur Bildung eines Ionenpaares eingesetzt. Ionenpaare sind physikochemische Molekularassoziate, die aus zwei geladenen Molekülen bestehen, welche durch eine wirksame Neutralisierung der ionischen Verhältnisse ein nach außen nahezu neutral erscheinendes und damit lipophileres Assoziat bilden. Somit wird der Wirkstoff, der jedesmal ein Teil dieses Assoziates ist, in eine lipophilere, diffusiblere und damit für die Hautpermeation besser geeignete Form überführt. Solche Assoziate können aus diesem Grunde auch die Kombination zweier Wirkstoffe darstellen, wie z. B. Morphin und Diclofenac. Zahlreiche Patentliteratur und auch einige Marktprodukte machen von diesem Prinzip Gebrauch. Beispielhaft sind zu nennen: EP 305 726B und EP 792 145 B

Die Internationale Patentveröffentlichung WO 02/41878 beschreibt ein transdermales und therapeutisches System vom Matrix-Typ. Es umfasst eine wirkstoffundurchlässige Rückschicht, eine wirkstoffhaltige Matrix auf Basis von Polysiloxan-Polymethacyrlat-Copolymeren und eine optionale Klebeschicht. Der Wirkstoff kann ausgewählt werden aus Fentanyl, Salbutamol oder Memantine sowie Diclofenac. Als Trägermaterial oder auch als Permeationsenhancer kommen Fettsäuren zum Einsatz.

Die Problematik der Anwendung solcher Ionenpaare innerhalb von transdermalen Systemen liegt insbesondere darin, dass sowohl pharmazeutische Wirkstoffe wie auch die als Zusatzstoffe dienenden Ionenpaarbildner die Kohäsion der Grundpolymere verringern und damit die Klebkraft verschlechtern können. Eine Abhilfe ist zwar dadurch möglich, daß sowohl die Konzentration des Ionenpaarbildners (Zusatzstoff) als auch die des pharmazeutischen Wirkstoffs herabgesetzt wird. Dann nimmt aber die thermodynamische Aktivität, welche den Wirkstofffluß bedingt, ab, kommt nicht voll zum Tragen und der Wirkstofffluß bleibt niedrig.

Eine sich aus diesem Stand der Technik ableitende Aufgabenstellung der vorliegenden Erfindung liegt also in der Bereitstellung einer Matrix für transdermale Systeme, welche Ionenpaare aus ladungsfähigen Wirkstoffen und physiologisch akzeptablen gegensinnig ionenpaarbildenden Zusatzstoffen enthält, wobei diese Matrix die Nachteile unzureichender Wirkstoffsättigung bzw. nachteiliger Weichmachung der Polymergrundlage nicht aufweist.

Unabhängig von dieser Aufgabenstellung sind seit einiger Zeit Silikone und Silikonklebmassen als Grundbestandteile von transdermalen Matrices bekannt und werden insbesondere wegen ihrer guten Verträglichkeit eingesetzt. Jedoch weisen diese üblicherweise geringe Löslichkeiten für Wirkstoffe bei hoher Diffusibilität auf. Wegen ihres ungünstigen Lösungsverhaltens wurden sie als Dispergiermedium für so genannte Mikroreservoire eingesetzt, in denen "ambiphile" Lösungsmittel für den Wirkstoff enthalten sind (EP 1 191 927 B). Gemäß dieser Veröffentlichung lösen diese ambiphilen Lösungsmittel den Wirkstoff während der Lagerung so, daß er nicht auskristallisiert, verlassen nach dem Aufkleben des transdermalen therapeutischen Systems die Matrix durch Einwandern in die Haut und hinterlassen anschließend eine Übersättigung mit nachfolgend erhöhter thermodynamischer Aktivität des Wirkstoffes. Ionenpaare und Ionenpaarbildner sind nicht Gegenstand dieser Veröffentlichung.

Eine vergleichbare Veröffentlichung (DE 101 41 651 A1) betrifft Systeme zur transdermalen Abgabe von Fentanyl und Fentanylderivaten, welche Silikonmatrices mit darin befindlichen Mikroservoiren umfassen, worin Lösungsmittel als leichtdiffusible Neutralstoffe wie z.B. Dipropylenglycol oder 1,3-Butandiol enthalten sind. In der allgemeinen Beschreibung von transdermalen therapeutischen Systemen, die mit diesen Matrices hergestellt werden, finden sich Hinweise darauf, daß Permeationsverstärker als weitere Inhaltsstoffe benutzt werden können. In dieser Aufzählung finden sich neben Glycerinestern, Fettalkoholen und Fettsäureestern auch Fettsäuren ohne Hinweis, wo diese innerhalb der transdermalen therapeutischen Systeme in welcher Zustandsform untergebracht werden sollen.
Die Verwendung von kationeriaktiven Ionenpaarbildnern und anionenaktiven Ionenpaarbildnern, wobei einer dieser genannten Stoffe ein pharmazeutischer Wirkstoff und der andere ein Zusatzstoff ist, in wässrigen Mikroemulsionen für eine nasale, rektale und/oder transdermale Applikation wird in DE 39 08 047 C2 beschrieben.

Der vorstehend genannte Stand der Technik löst aber nicht die erfindungsgemäße Aufgabe, Ionenpaare bestehend aus Wirkstoffen und komplementär geladenen Zusatzstoffen mit hoher thermodynamischer Aktivität und geringstmöglicher Beeinflussung des polymeren Gefüges in transdermalen Systemen, welche mindestens eine Polymermatrix enthalten, zur Verfügung zu stellen.

Die Lösung der erfindungsgemäßen Aufgabe besteht in der Bereitstellung eines transdermalen therapeutischen Systems umfassend als mindestens eine Systemkomponente eine Matrix, bestehend aus einem Polymer-Grundmaterial aus Polysiloxan, welches Mikroreservoire enthält, **dadurch gekennzeichnet, daß** in den genannten Mikroreservoiren der Wirkstoff in der Form eines Ionenpaares bestehend aus einem ladungsfähigen Wirkstoff und einem gegenläufig ladungsfähigen physiologisch unbedenklichen Zusatzstoff ohne die Gegenwart von Lösungsmitteln enthalten ist, wobei dieser zur Ionenpaarbildung befähigte Zusatzstoff in mindestens äquimolarer Menge anwesend ist.

Die Erfindung wird im Folgenden weiter beschrieben. Geeignete Wirkstoff-Ionenpaare können grundsätzlich in der Paarung aus zunächst basisch vorliegendem, also nucleophil reagierendem Wirkstoffteil mit mindestens äquimolar zugesetztem sauerem, also elektrophil reagierenden Zusatzstoffteil bestehen.
Hierzu geeignete Wirkstoffe sind z.B. Nikotin, Moxonidin, Clonidin, Scopolamin, Atropin, Buprenorphin, Fentanyl, Salbutamol, Memantine, u.v.a. hochwirksame, insbesondere aminische Wirkstoffe. Besonders bevorzugt sind Wirkstoffe, deren Tagesdosis 30 mg nicht überschreitet, da aufgrund der begrenzten Hautpermeation sich hier besondere Vorteile ergeben. Geeignete elektrophile Zusatzstoffe zur Bildung eines Ionenpaares sind gesättigte und ungesättigte Fettsäuren, die verzweigt oder unverzweigt unmodifiziert oder hydroxyliert (Rizinolsäure, Levulinsäure) oder auch auf sonstige Weise derivatisiert sind. Durchaus geeignet sind aber auch aromatische Verbindungen, sofern sie auch die Fähigkeit, kationische Ionenpaarkomplexe mit Wirkstoffen aufzubauen, aufweisen. Beispielhaft seien hier noch Benzoesäure, Heptansulfonsäure, aber auch Zitronensäure, Weinsäure und sogar anorganische Säuren, wie Phosphorsäure oder Salzsäure genannt. Da die Effizienz der Ionenpaarbildung für die Begünstigung der transdermalen Resorption in besonderer Weise durch lyophile Säuren zu erreichen ist, sind Säurezusatzstoffe bevorzugt, welche einen Octanol/Puffer (pH 5,5 Verteilungskoeffizienten von mehr als 1) aufweisen.

In analoger Weise ist Gegenstand dieser Erfindung ein transdermales therapeutisches System, daß dadurch gekennzeichnet ist, daß in den Mikroreservoiren Ionenpaare aus sauren, d.h. elektrophilen Wirkstoffen mit der Befähigung zur anionischen Ladung und basischen, d.h. nucleophil reagierenden Zusatzstoffen mit der Befähigung zur kationischen Ladung, die in mindestens äquimolarer Menge zugegen sind, vorliegen. Beispiele für saure, d.h. elektrophil reagierenden Wirkstoffe sind Diclofenac, Ketoprofen, Ibuprofen, Acetylsalicylsäure, Salicylsäure und viele andere Wirkstoffe mit Bevorzugung solcher, die eine systemische Tagesdosis von weniger als 30 mg aufweisen. Geeignete Zusatzstoffe für solche Ionenpaare sind alle Amine, bevorzugt Diethanolamin, Triethanolamin, Dimethylaminoethanol, Tromethamol und jede andere, physiologisch verträgliche Substanz, welche als Base zur Bildung von Ionenpaaren mit anionischen Wirkstoffen in der Lage ist.

Die erfindungsgemäßen Ionenpaare werden in einer diskreten physikalischen Phase als halbfest/feste oder flüssige Innenphase innerhalb der Matrix eingebettet, die im wesentlichen aus Polysiloxanen besteht. Die Matrix und auch die Innenphase mit dem Ionenpaar kann weitere Stoffe, wie z.B. Enhancer, Weichmacher, hydrophile oder lipophile Polymere etc. enthalten. Besonders bevorzugt sind aber erfindungsgemäß Matrices, deren Innenphase ausschließlich aus dem Ionenpaar ("physikalisch/chemisches Gemisch" des Wirkstoffes und mindestens eines in äquimolarer Menge zugesetzten Ionenpaarbilderns) besteht.

Auf diese Weise ist die erfindungsgemäße Wirkung, nämlich die Verringerung der Auswirkung auf die Kohäsion der umgebenden Silikonmatrix und auf der anderen Seite die maximale Erhöhung der Sättigung (thermodynamischen Aktivität), des Wirkstoffes (Wirkstoff-lonenpaars) erreichbar.

Die erfindungsgemäße Matrix kann im einfachsten Falle mit einer in der Regel wirkstoffundurchlässigen, die Verklebung mit Textilien vermeidenden Rückschicht ausgestattet werden, für die sich insbesondere Polyester-Materialien eignen. Die Matrix kann, wenn die verwendeten Silikonpolymere Silikonklebemassen sind, als Direktverklebung auf die Haut ausgestattet werden. Weitere Lösungen bestehen in einer Kombination mit kontrollierenden Membranen, weiteren Klebschichten oder Reservoirschichten, wie sie dem Fachmann bekannt sind.

Die Herstellung des erfindungsgemäßen Systems kann auf verschiedenartige Weise erfolgen, auch aufbauend auf zahlreiche, dem Fachmann bekannte Verfahren des Erzeugens von Innenphasen in Matrixsystemen. Beispielhaft und bevorzugt werden für die vorgelegten erfindungsgemäßen Rezepturen folgende Prozesse angewendet:

Das zur Matrix-Außenphase vorgesehene Polysiloxan oder Polysiloxan-Derivat wird zur Erreichung der Fließfähigkeit mit einem bei Raumtemperatur flüchtigen, in der Regel mit Wasser nicht mischbaren Lösemittel versetzt, und so eine streichbare Masse erhalten. "Anionenbildner" und "Kationenbildner" werden zugesetzt und es wird unter intensiver Scherung und Rührung eine Vereinigung der immer feiner dispergierten Einzelpartikel zu Einzeltröpfchen erreicht, die auf eine Reaktion der beiden Zusätze zurückzuführen ist. Die Masse wird anschließend in einer dem transdermalen therapeutischen System angemessenen Schichtdicke auf eine geeignete dehäsiv ausgerüstete Folie beschichtet und unter Wärmeanwendung so getrocknet, daß das meist lipophil gewählte Lösemittel bis auf Spuren vollständig entfernt wird.

Eine Abwandlung dieser Technik beruht darin, das schon in Lösung befindliche Polysiloxan oder Polysiloxan-Derivat mit einer Vorlösung aus einem weiteren Lösemittel, dem Wirkstoff und einer mindestens äquimolar zugesetzten Menge des Gegenionbildners nach vorheriger kompletter Auflösung zu versetzen. Anschließend wird intensiv gerührt, so daß die später zugesetzte Innenphase homogen verteilt ist.

Die Herstellung der erfindungsgemäßen transdermalen therapeutischen Systeme wird durch die nachfolgenden Beispiele näher erläutert:

### Beispiel 1:

2 g Moxonidin und 10 g Laurinsäure werden in 50 g absolutem Ethanol vollständig unter leichtem Erwärmen aufgelöst. Diese Lösung wird einer vorher bereiteten Lösung von 200 g festem Polysiloxan in 200 g n-Heptan zugesetzt und mit einem Rührflügelrührer 10 min. intensiv gerührt. Die zweiphasige Lösung wird unter Rühren mit einer Schichtdicke von ca. 50 µm auf eine mit Fluorpolymer oberflächenbehandelte PET-Folie beschichtet und für drei Minuten bei Raumtemperatur getrocknet. Das Produkt stellt eine erfindungsgemäße Matrix dar, welche sich für die weitere Verarbeitung zu transdermalen therapeutischen Systemen eignet.

### Beispiel 2:

Die gemäß Beispiel 1 gefertigte Masse wird mit 50 g mikronisiertem und quervernetztem Polyvinylpyrrolidon versetzt und noch für etwa fünf Minuten weiter gerührt. Unter diesen Umständen bildet sich eine von der Korngröße her durch die zugesetzten quellbaren Polyvinylpyrrolidon-Partikel bestimmte Innenphase-Mikroreservoir-Größe aus, die auch nach dem Beschichten und Trocknen, die analog Beispiel 1 erfolgt, bestehen bleibt.

## Patentansprüche

1. Transdermales therapeutisches System, umfassend eine wirkstoffundurchlässige Rückschicht, mindestens eine aus Polysiloxanen und/oder Polysiloxan-Derivaten bestehende Matrixschicht enthaltend Mikroservoire, sowie eine abziehbare, wirkstoffundurchlässige Schutzschicht, **dadurch gekennzeichnet, daß** die Mikroreservoire innerhalb der Matrix eine feste, halbfeste oder flüssige Innenphase bilden, welche aus mindestens einem Ionenpaar aus jeweils einem pharmakologisch aktiven Wirkstoff und einem Zusatzstoff besteht, wobei der Zusatzstoff in mindestens aquimolarer Menge vorliegt und entweder der Wirkstoff ein Kationenbildner und der Zusatzstoff ein Anionenbildner oder der Wirkstoff ein Anionenbildner und der Zusatzstoff ein Kationenbildner ist.

2. Transdermales therapeutisches System gemäß Anspruch 1, **dadurch gekennzeichnet, dass** das Ionenpaar in Form eines physikochemischen Molekularassoziats vorliegt.

3. Transdermales therapeutisches System gemäß Anspruch 1, **dadurch gekennzeichnet, dass** die Innenphase mindestens 0,2 Gew.-% der Matrix darstellt.

4. Transdermales therapeutisches System gemäß Anspruch 1, **dadurch gekennzeichnet, dass** es mindestens eine Membranschicht enthält.

5. Transdermales therapeutisches System gemäß Anspruch 1, **dadurch gekennzeichnet, dass** es eine zusätzliche Klebschicht enthält.

6. Transdermales therapeutisches System gemäß den Ansprüchen 1-5, **dadurch gekennzeichnet, dass** als kationenbildende Wirkstoffe Nikotin, Moxonidin, Clonidin, Scopolamin, Atropin, Buprenorphin, Fentanyl sowie dessen Derivate, Salbutamol oder Memantine verwendet werden.

7. Transdermales therapeutisches System gemäß den Ansprüchen 1-5, **dadurch gekennzeichnet, dass** als anionenbildende Wirkstoffe Diclofenac, Ketoprofen, Ibuprofen, Acetylsalicylsäure oder Salicylsäure verwendet werden.

8. Transdermales therapeutisches System gemäß mindestens einem der vorhergehenden Ansprüche, **dadurch gekennzeichnet, dass** als anionenbildende Zusatzstoffe gesättigte oder ungesättigte Fettsäuren oder deren Derivate verwendet werden.

9. Transdermales therapeutisches System gemäß mindestens einem der vorhergehenden Ansprüche, **dadurch gekennzeichnet, dass** als kationenbildende Zusatzstoffe Amine verwendet werden.

10. Transdermales therapeutisches System gemäß Anspruch 9, **dadurch gekennzeichnet, daß** Diethanolamin, Triethanolamin, Dimethylaminoethanol und/oder Tromethanol verwendet werden.

11. Transdermales therapeutisches System gemäß mindestens einem der vorhergehenden Ansprüche, **dadurch gekennzeichnet, dass** die Matrixschicht Permeationsenhancer, Weichmacher und/oder hydrophile oder lipophile Polymere enthält.

## Claims

1. A transdermal therapeutic system comprising an active ingredient-impermeable backing layer, at least one matrix layer consisting of polysiloxanes and/or polysiloxane derivatives and comprising microreservoirs, and a detachable, active ingredient-impermeable protective layer, **characterized in that** the microreservoirs within the matrix form a solid, semisolid or liquid internal phase which consists of at least one ion pair of in each case one pharmacologically active ingredient and one additive, where the additive is present in at least equimolar quantity, and either the active ingredient is a cation former and the additive is an anion former or the active ingredient is an anion former and the additive is a cation former.

2. The transdermal therapeutic system as claimed in claim 1, **characterized in that** the ion pair is present in the form of a physicochemical molecular association.

3. The transdermal therapeutic system as claimed in claim 1, **characterized in that** the internal phase represents at least 0.2% by weight of the matrix.

4. The transdermal therapeutic system as claimed in claim 1, **characterized in that** it comprises at least one membrane layer.

5. The transdermal therapeutic system as claimed in claim 1, **characterized in that** it comprises an additional adhesive layer.

6. The transdermal therapeutic system as claimed in claims 1-5, **characterized in that** nicotine, moxonidine, clonidine, scopolamine, atropine, buprenorphine, fentanyl and its derivatives, salbutamol or memantine are used as cation-forming active ingredients.

7. The transdermal therapeutic system as claimed in claims 1-5, **characterized in that** diclofenac, ketoprofen, ibuprofen, acetylsalicylic acid or salicylic acid are used as anion-forming active ingredients.

8. The transdermal therapeutic system as claimed in at least one of the preceding claims, **characterized in that** saturated or unsaturated fatty acids or derivatives thereof are used as anion-forming additives.

9. The transdermal therapeutic system as claimed in at least one of the preceding claims, **characterized in that** amines are used as cation-forming additives.

10. The transdermal therapeutic system as claimed in claim 9, **characterized in that** diethanolamine, triethanolamine, dimethylaminoethanol and/or tromethanol are used.

11. The transdermal therapeutic system as claimed in at least one of the preceding claims, **characterized in that** the matrix layer comprises permeation enhancers, plasticizers and/or hydrophilic or lipophilic polymers.

## Revendications

1. Système thérapeutique transdermique, comprenant une couche de dos imperméable aux substances actives, au moins une couche de matrice consistant en un polysiloxane et/ou en dérivés de polysiloxane, ainsi qu'une couche protectrice détachable, imperméable aux substances actives, **caractérisé en ce que** les microréservoirs à l'intérieur de la matrice forment une phase interne solide, semi-solide ou liquide, qui est constituée d'au moins une paire d'ions provenant chacun d'une substance pharmacologiquement active et d'un additif, l'additif étant présent en quantité au moins équimolaire et soit la substance active est un générateur de cation et l'additif est un générateur d'anion, soit la substance active est un générateur d'anion et l'additif est un générateur de cation.

2. Système thérapeutique transdermique selon la revendication 1, **caractérisé en ce que** la paire d'ions se trouve sous forme d'une association moléculaire physico-chimique.

3. Système thérapeutique transdermique selon la revendication 1, **caractérisé en ce que** la phase interne représente au moins 0,2 % en poids de la matrice.

4. Système thérapeutique transdermique selon la revendication 1, **caractérisé en ce qu'**il contient au moins une couche membranaire.

5. Système thérapeutique transdermique selon la revendication 1, **caractérisé en ce qu'**il contient une couche adhésive supplémentaire.

6. Système thérapeutique transdermique selon les revendications 1-5, **caractérisé en ce qu'**on utilise comme substances actives génératrices de cations la nicotine, la monoxidine, la clonidine, la scopolamine, l'atropine, la buprénorphine, le fentanyl ainsi que ses dérivés, le salbutamol et la mémantine

7. Système thérapeutique transdermique selon les revendications 1-5, **caractérisé en ce qu'**on utilise comme substances actives génératrice d'anions le diclofénac, le kétoprofène, l'ibuprofène, l'acide acétylsalicylique ou l'acide salicylique.

8. Système thérapeutique transdermique selon au moins l'une des revendications précédentes, **caractérisé en ce qu'**on utilise comme additifs générateurs d'anions des acides gras saturés ou insaturés ou leurs dérivés.

9. Système thérapeutique transdermique selon au moins l'une des revendications précédentes, **caractérisé en ce qu'**on utilise comme additifs générateurs de cations des amines.

10. Système thérapeutique transdermique selon la revendication 9, **caractérisé en ce qu'**on utilise la diéthanolamine, la triéthanolamine, le diméthylaminoéthanol et/ou le trométhanol.

11. Système thérapeutique transdermique selon au moins l'une des revendications précédentes, **caractérisé en ce que** la couche de matrice contient des promoteurs de perméation, des plastifiants et/ou des polymères hydrophiles ou lipophiles.
